(19) Europäisches Patentamt
European Patent Office
Office européen des brevets

(11) Publication number : **0 539 180 A1**

# (12) EUROPEAN PATENT APPLICATION

(21) Application number : **92309622.6**

(22) Date of filing : **21.10.92**

(51) Int. Cl.$^5$ : **A61K 31/495,** A61K 31/445

(30) Priority : **25.10.91 JP 306597/91**

(43) Date of publication of application :
**28.04.93 Bulletin 93/17**

(84) Designated Contracting States :
**AT BE CH DE DK ES FR GB GR IT LI LU NL SE**

(71) Applicant : **SANWA KAGAKU KENKYUSHO CO., LTD.**
**No. 35, Higashi-sotobori-cho**
**Higashi-ku Nagoya-shi Aichi-ken (JP)**

(72) Inventor : **Kurono, Masayasu, Sanwa Kagaku Kenkyusho**
**Co., Limited, Higashi-sotobori-cho 35, Higashi-ku**
**Nagoya-shi, Aichi-ken (JP)**
Inventor : **Ishiwata, Yoshiro, Sanwa Kagaku Kenkyusho**
**Co., Limited, Higashi-sotobori-cho 35, Higashi-ku**
**Nagoya-shi, Aichi-ken (JP)**
Inventor : **Yokochi, Shiyojio, Sanwa Kagaku Kenkyusho**
**Co., Limited, Higashi-sotobori-cho 35, Higashi-ku**
**Nagoya-shi, Aichi-ken (JP)**
Inventor : **Asano, Kyoichi, Sanwa Kagaku Kenkyusho**
**Co., Limited, Higashi-sotobori-cho 35, Higashi-ku**
**Nagoya-shi, Aichi-ken (JP)**
Inventor : **Mitani, Takahiko, Sanwa Kagaku Kenkyusho**
**Co., Limited, Higashi-sotobori-cho 35, Higashi-ku**
**Nagoya-shi, Aichi-ken (JP)**
Inventor : **Kakigami, Takuji, Sanwa Kagaku Kenkyusho**
**Co., Limited, Higashi-sotobori-cho 35, Higashi-ku**
**Nagoya-shi, Aichi-ken (JP)**
Inventor : **Iwata, Noriyuki, Sanwa Kagaku Kenkyusho**
**Co., Limited, Higashi-sotobori-cho 35, Higashi-ku**
**Nagoya-shi, Aichi-ken (JP)**
Inventor : **Isogawa, Kougaku, Sanwa Kagaku Kenkyusho**
**Co., Limited, Higashi-sotobori-cho 35, Higashi-ku**
**Nagoya-shi, Aichi-ken (JP)**
Inventor : **Baba, Yutaka, Sanwa Kagaku Kenkyusho**
**Co., Limited, Higashi-sotobori-cho 35, Higashi-ku**
**Nagoya-shi, Aichi-ken (JP)**
Inventor : **Ohwaki, Hiroyuki, Sanwa Kagaku Kenkyusho**
**Co., Limited, Higashi-sotobori-cho 35, Higashi-ku**
**Nagoya-shi, Aichi-ken (JP)**
Inventor : **Sawai, Kiichi, Sanwa Kagaku Kenkyusho**
**Co., Limited, Higashi-sotobori-cho 35, Higashi-ku**
**Nagoya-shi, Aichi-ken (JP)**

(74) Representative : **Bubb, Antony John Allen et al**
**GEE & CO. Chancery House Chancery Lane**
**London WC2A 1QU (GB)**

## (54) Antiviral agents.

(57) Antiviral compounds (I) :

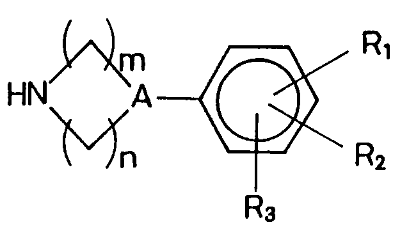

(I)

the symbols being as defined in the specification, are efficacious against infections caused by a variety

EP 0 539 180 A1

of DNA viruses, RNA viruses and retroviruses. Other specified compounds also exhibit activity. The compounds have a wider spectrum of activity than known antiviral substances.

The present invention concerns medicaments for preventing and treating infections caused by a variety of DNA viruses, RNA viruses and retroviruses.

So far, modified or unmodified phenylpiperazine, phenylpiperidine, phenylpyrrolidine, tetrahydroimidazole and phenylhomopiperazine have been utilized as effective substituents in producing numerous pharmaceuticals. However, antivirally active agents usually have these compounds as basic skeletons and derive their activities from the compounds themselves.

On the other hand, well-known antivirally active agents containing these compounds as substituents include glycyrrhetinic acid derivatives (JP-A-3-127271 laid open for public inspection) discovered by us, pyridazine derivatives (EP-A-1564333 and JP-A-60-226862 laid open for public inspection), and imidazole analogs ("Antimicrobial Agents and Chemotherapy", Vol. 21(2), pp. 358-361 (1982)), but the number thereof is very limited.

Compounds containing modified or unmodified phenylpiperazine, phenylpiperidine, phenylpyrrolidine, tetrahydroimidazole or phenylhomopiperazine as substituents and which have turned out to be active against herpes viruses as well are limited to glycyrrhetinic acid derivatives (JP-A-3-127271 laid open for public inspection) - discovered by us - and thymidine analogs ("Antimicrobial Agents and Chemotherapy", Vol. 21(2), pp. 358-361 (1982)).

On the other hand, pyridazine derivatives containing modified or unmodified phenylpiperazine, phenylpiperidine, phenylpyrrolidine, tetrahydroimidazole or phenylhomopiperazine as substituents (EP-A-156433 and JP-A-60-226862 laid open for public inspection) are known to be active against Picornaviridae viruses represented by rhinoviruses, but not against herpes viruses.

Furthermore, compounds containing modified or unmodified phenylpiperazine, phenylpiperidine, phenylpyrrolidine, tetrahydroimidazole or phenylhomopiperazine as substituents and effective against both herpes viruses and influenza viruses are limited to the glycyrrhetinic acid derivatives (JP-A-3-127271 laid open for public inspection).

In recent years, the research and development of antiviral agents have been remarkable. A nucleic acid type of antiviral agent, represented by acyclovir in particular, has proved to be clinically efficacious against infection caused by herpes simplex viruses and zoster viruses. However, another problem has arisen in connection with infections with resistance to herpes viruses, for instance, thymidine kinase negative types ("Oral. Surg. Oral. Med. Oral. Pathol.", Vol. 67, pp. 427-432 (1989)). These nucleic acid type antiviral drugs are also generally known to be inefficacious against RNA viruses represented by influenza viruses.

Among antiviral agents other than the nucleic acid type antiviral agents and efficacious against DNA viruses as well there are known, for instance, phosphonoformate (PFA for short; see "Nippon Rinsho", Vol. 47(2), pp. 390-394 (1989)), phosphonoacetate (PAA for short; see "Nippon Rinsho", Vol. 47(2), pp. 390-394 (1989)), and cycloxolone ("Journal of Antimicrobial Chemotherapy", Vol. 18, Suppl. B. pp. 185-200 (1986) are known. However, problems with phosphonoformate and phosphonoacetate are that they have side effects such as renal disorders and anemia, whereas cycloxolone has insufficient antiviral activity for use in therapy.

Among a group of antiviral agents other than the nucleic acid type antiviral agents and efficacious against RNA viruses as well are, for instance, amantadine ("Shonika Sinryo", Vol. 54(4), pp 988-994 (1991)), rimantadine ("Shonika Sinryo", Vol. 54(4), pp. 988-994 (1991)) and LY253963 (a thiadiazol derivative; ("Shonika Sinryo", Vol. 54(4), pp. 988-994 (1991)). Amantadine and rimantadine have been reported to be efficacious against influenza A viruses but not against influenza B viruses, and pose a grave problem - a side effect on the central nerve. LY253963 has been shown to be resistant to influenza viruses in animal tests, but its clinical efficacy has yet to be verified.

For instance, AZT and DDI are known as antiviral agents that are efficacious against retroviruses represented by AIDS viruses (HIV) ("Shonika Sinryo", Vol. 54 (4), pp. 981-987 (1991)). Although these agents delay the development of AIDS, they cannot accelerate healing and have severe side effects such as myelopathy.

In recent years, patients with immunodeficiency diseases induced by organ transplantation, cancer chemotherapy and HIV infections have been on the increase, posing a grave medical problem. Virus infections in such patients are so diverse in type that they cannot often be treated with existing antiviral drugs. Much is thus now expected from the development of more greatly improved antiviral drugs, and of more efficacious antiviral drugs for HIV and HCV as well.

It is therefore a primary object of the invention to provide a novel antiviral agent that has an excellent effect on DNA viruses, RNA viruses and retroviruses. Another object of the invention is to provide a novel antiviral agent that is different in chemical structure from conventional antiviral agents and so will be efficacious against viruses resistant to conventional antiviral substances.

We have already revealed that some compounds having one of various substituents at the 30-position of glycyrrhetinic acid have an excellent antiviral activity (see JP-A-3-127271 laid open for public inspection). Among them, a compound having a phenylpiperazine derivative at the 30-position of glycyrrhetinic acid, i.e.,

1-[3β-(3-carboxy-propanoyloxy)-18β-olean-12-en-30-yl]-4-(o-methoxyphenyl) piperazine, has been found to have a particularly high antiviral activity and to be very safe.

We have studied the antiviral and cytotoxic actions of modified or unmodified phenylpiperazine, phenylpiperidine, phenylpyrrolidine, tetrahydroimidazole and phenylhomopiperazine, all known in the art. As a result, we have discovered that a number of compounds have a wide spectrum of antiviral actions on various DNA viruses, RNA viruses and retroviruses, like herpes simplex viruses (types 1 and 2), vaccinia viruses, zoster viruses, cytomegaloviruses, influenza viruses, hepatitis B viruses and AIDS viruses (HIV), and are efficacious for both topical and systemic therapies.

We then investigated known, modified or unmodified phenylaziridine, phenylazetidine, phenyldiazetidine and phenylperhydroazepine that are similar in chemical structure to phenylpiperazine, phenylpiperidine, phenylpyrrolidine, tetrahydroimidazole and phenylhomopiperazine, and have consequently found out that some of these compounds have excellent antiviral action.

After we had discovered the antiviral actions of phenylpiperazine, phenylpiperidine, phenylpyrrolidine, tetrahydroimidazole and phenylhomopiperazine, as well as modified or unmodified phenylaziridine, phenylazetidine, phenyldiazetidine and phenylperhydroazepine, we studied the antiviral activities of compounds having these compounds in their structures as substituents. Interestingly enough, we discovered that some of these compounds have strong antiviral activity.

According to our findings as mentioned above, it is possible to provide a novel, more efficacious antiviral agent by making a variety of modifications to the basic skeletons comprising phenylpiperazine, phenylpiperidine, phenylpyrrolidine, tetrahydroimidazole, phenylhomopiperazine, phenylaziridine, phenylazetidine, phenyldiazetidine and phenylperhydroazepine.

It is also possible to endow some known compounds with antiviral activity to enhance their antiviral activity by introducing thereinto modified or unmodified phenylpiperazine, phenylpiperidine, phenylpyrrolidine, tetrahydroimidazole, phenylhomopiperazine, phenylaziridine, phenylazetidine, phenyldiazetidine and phenylperhydroazepine.

Mentioned as compounds into which modified or unmodified phenylpiperazine, phenylpiperidine, phenylpyrrolidine, tetrahydroimidazole, phenylhomopiperazine, phenylaziridine, phenylazetidine, phenyldiazetidine and phenylperhydroazepine can be introduced, are substances that occur naturally or in vivo such as nucleic acid types of antiviral substances represented by acyclovir and Ara-A; non-steroidal, anti-inflammatory substances used in combination with the treatment of virus infections;
antimicrobial/antibiotic substances such as sulfanilamide and its derivatives, β-lactam and pyridone carboxylate; fatty acids; amino acids; peptides; steroids; and flavones. It is noted, however, that the invention is not specifically limited to the substances mentioned above.

The phenylpiperazine, phenylpiperidine, phenylpyrrolidine, tetrahydroimidazole, phenylhomopiperazine, phenylaziridine, phenylazetidine, phenyldiazetidine and phenylperhydroazepine derivatives that are an effective component of the invention include known compounds represented by the general formula (I):

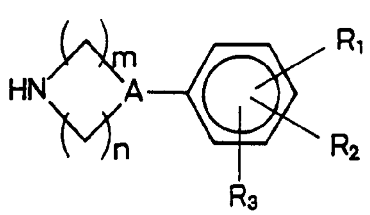

(I)

where:

$R_1$, $R_2$ and $R_3$ simultaneously or independently stand for a hydrogen atom, an amino group, an alkylamino group that may have a substituent, an acylamino group, an alkyl group that may have a substituent, a hydroxy group, an alkyloxy group that many have a substituent, a halogeno group, a carboxy group, an alkylcarbonyl group that may have a substituent, an alkoxycarbonyl group that may have a substituent, a carbamoyl group that may have a substituent, a nitro group, a cyano group, a thiol group, an alkylthio group that may have a substituent, a phenyl group that may have a substituent and a heterocyclic ring;

A denotes a nitrogen atom or a methyne group;

m indicates 0 or a natural number; and

n means a natural number;

or its salt. In this connection, it is to be understood that the term "that may have a substituent" means that the group may be substituted as by an amino, halogeno, nitro, cyano, thiol, acyl or carbamoyl group, and the term "heterocyclic ring" means, for instance, pyridine, piperidine, pyrazine, pyrrole, pyrrolidine, oxazole, imidazole, morpholine, diazole, tetrazole, thiazole or thiadiazole.

Compounds that are not represented by the general formula (I) mentioned above but can be used as an effective component in the invention, for instance, include 4-(2-aminoethyl)-1-(o-methoxyphenyl)piperazine, (3α,16α)-eburnamonine-O-[2-[4-(o-methoxyphenyl)-1-piperazinyl]ethyl]oxime (JP-A-61-12687 laid open for public inspection) and (3α,16α)-eburnamonine-O-[2-hydroxy-3-[4-(o-methoxyphenyl)-1-piperazinyl]ethyl]oxime (JP-A-61-12687 laid open for public inspection), and their salts.

Specific, but not exclusive, examples of the phenylpiperazine, phenylpiperidine, phenylpyrrolidine, tetrahydroimidazole, phenylhomopiperazine, phenylaziridine, phenylazetidine, phenyldiazetidine and phenylperhydroazepine derivatives represented by the general formula (I) are:

1-phenylpiperazine,
1-(o-chlorophenyl)piperazine,
1-(m-chlorophenyl)piperazine,
1-(p-chlorophenyl)piperazine,
1-(o-fluorophenyl)piperazine,
1-(m-fluorophenyl)piperazine,
1-(p-fluorophenyl)piperazine,
1-(o-methoxyphenyl)piperazine,
1-(m-methoxyphenyl)piperazine,
1-(p-methoxyphenyl)piperazine,
1-(o-methylphenyl)piperazine,
1-(m-methylphenyl)piperazine,
1-(p-methylphenyl)piperazine,
1-(o-trifluoromethylphenyl)piperazine,
1-(m-trifluoromethylphenyl)piperazine,
1-(p-trifluoromethylphenyl)piperazine,
1-(o-nitrophenyl)piperazine,
1-(m-nitrophenyl)piperazine,
1-(p-nitrophenyl)piperazine,
1-(2,6-dichlorophenyl)piperazine,
or 1-phenylpiperidine, and their salts.

The phenylpiperazine, phenylpiperidine, phenylpyrrolidine, tetrahydroimidazole, phenylhomopiperazine, phenylaziridine, phenylazetidine, phenyldiazetidine and phenylperhydroazepine derivatives represented by the general formula (I) as well as 4-(2-aminoethyl)-1-(o-methoxyphenyl)piperazine, (3α,16α)-ebrunamonine-O-[2-[4-(o-methoxyphenyl)-1-piperazinyl]ethyl]oxime and (3α,16α)-eburnamonine-O-[2-hydroxy-3-[4-(o-methoxyphenyl)-1-piperazinyl]propyl]oxime have strong antiviral activities and low toxicities, and may be provided in the form of various preparations.

For topical therapy, the antiviral agent of the invention may be used in the form of liquid, ointment, cream, gel and suppository (for the rectum and the vagina) preparations for the skin and mucosae, and in the form of eye lotion and ointment preparations. It is noted that these preparations may be obtained in conventional manner. When administered to human patients, it is generally preferable to use preparations containing 0.01 to 10% of the compound according to the invention, although varying depending on what type of compound is used, what condition the patients are in and what preparation of compound is administered.

In connection with promoting absorption, it is noted that such absorption promoters as bile acids, saponins, polyoxyethylene higher alcohol ether, polyethylene glycol, DMSO and laurolactam may be added to the preparations of the invention, as desired.

The antiviral agent of the invention, when administered orally to human patients, may be used in the form of liquids, tablets, capsules, granules, grains, buccal tables and troches, which may again be obtained conventionally. In connection with promoting absorption, it is noted that such absorption promoters as bile acids, saponins and polyoxyethylene higher alkyl ether may be added to the preparations, as desired. When administered to human patients, these preparations may suitably and generally be used in doses of 100 to 3,000 mg/day, although varying depending on what type of compound is used, what conditions the patients are in and what preparation of compound is administered. Injections may also be obtained conventionally, and may generally be administered human patients in a dose of 30 to 1,000 mg/day, although depending on what type of compound is used, what condition the patients are in, and what preparation of compound is administered.

## EXAMPLES

### PHARMACOLOGICAL EFFICACY TESTING - 1

### In Vitro Antiviral Activities Against Herpes Simplex Vituses Type 1 and Cytotoxicity

Confluent GMK cells (derived from a green monkey's kidney) in 96-well plates were infected with HSV-1 (Miyama strain) in the presence of various concentrations of test compounds. After incubation, virus-induced cytopathic effect (CPE), inhibitory effect of CPE by the test compounds, and cytotoxicity were microscopically observed. The virus titer ($TCID_{50}$) was determined from CPE of virus infected culture. Antiviral effects of the test compounds were calculated from $TCID_{50}$ values of the test compounds treated and control cultures and represented by $\Delta TCID_{50}$ ($log_{10}$).

The results are shown in Table 1. In this regard, it is noted that prior to be added to the incubation systems, the compounds were dissolved in MEM or ethanol media at 10 mg/ml concentrations, and diluted with MEM media supplemented with 1% fetal bovine serum.

Table 1

In Vitro Action on HSV-1 (Miyama Strains)

| Test Compound Nos. | Antiviral Activity ($\Delta TCID_{50}$ ($log_{10}$)) | | |
|---|---|---|---|
| Concentrations ($\mu g/ml$) | 10 | 50 | 100 |
| Compound 1 | 0.56(-) | 1.90(-) | >3.56(±) |
| 2 | 0.46(-) | 1.23(-) | (±±) |
| 3 | 0.0 (-) | 1.50(-) | (±±) |
| 4 | 0.56(-) | 1.56(-) | 2.50(+) |
| 5 | 0.33(-) | 1.33(-) | 1.56(+) |
| 6 | 0.0 (-) | 0.50(-) | 1.33(+) |
| 7 | | | 0.83(+) |
| 8 | | | 0.50(+) |
| 9 | 0.87(-) | 1.80(-) | 2.53(-) |
| 10 | >3.00(-) | >2.17(±) | |
| 11 | 2.00(-) | >4.00(-) | <4.00(±) |
| 12 | 1.00(-) | 2.67(-) | >3.30(±) |

In Table 1, parentheses indicate the cytotoxicities of these compounds; (-), (±), (+) and (±±) indicate no cytoxicity, slight cytotoxicity, cytotoxicity with an observable antiviral effect and cytotoxicity with an undetectable antiviral effect, respectively.

Compound 1: 1-(o-chlorophenyl)piperazine hydrochlorate

2: 1-(m-chlorophenyl)piperazine hydrochlorate

3: 1-(p-chlorophenyl)piperazine hydrochlorate

4: 1-(o-methoxyphenyl)piperazine hydrochlorate

5: 1-(o-fluorophenyl)piperazine hydrochlorate

6: 1-(p-fluorophenyl)piperazine hydrochlorate

7: 1-phenylpiperazine

8: 1-phenylpiperidine

9: 4-(2-aminoethyl)-1-(o-methoxyphenyl)piperazine

10: (3α, 16α)-ebrunamonine-O-[2-hydroxy-3-[4-(o-methoxyphenyl)-1-piperazinyl]propyl]oxime trihydrochlorate

11: 1-(o-trifluoromethylphenyl)piperazine

12: 1-(2,6-dichlorophenyl)piperazine

PHARMACOLOGICAL EFFICATY TESTING - 2

Antiviral Spectrum of 1-(o-chlorophenyl)piperazine hydrochlorate

Monolayer cells in 96-well plates were infected with HSV-1 (KOS strain), HSV-2 (UW-268 strain), vaccinia viruses (D1E strains) or influenza viruses (A/PR/8 strains) in the presence of the test compound. After incubation, the virus-induced cytopathic effect (CPE), inhibitory effect of CPE by the test compound and cytotoxicity were microscopically observed. The virus titer ($TCID_{50}$) was determined from CPE of the virus-infected cultures. The antiviral effect of the test compound was calculated from $TCID_{50}$ values of the compound treated and control cultures. The results were represented by $\Delta TCID_{50}$ ($\log_{10}$). The host cells were MDCK cells for influenza virus and Vero cells for other viruses.

The results are shown in Table 2. In this regard, it is noted that prior to be added to the incubation systems, the compounds were dissolved in MEM or ethanol media at 10 mg/ml concentrations, and diluted with MEM media supplemented with 1% fetal bovine serum.

Table 2

Antiviral Spectrum of 1-(o-chlorophenyl)piperazine hydrochlorate

| Viruses | Antiviral Activity ($\Delta TCID_{50}$ ($\log_{10}$)) | | |
|---|---|---|---|
| Concentrations (μg/ml) | 10 | 50 | 100 |
| HSV-1 | 0.20(-) | 1.20(-) | >3.36(-) |
| HSV-2 | 0.77(-) | 1.27(-) | >3.30(-) |
| Vaccinian viruses | 0.50(-) | 1.17(-) | 2.50(-) |
| Influenza viruses | 1.26(-) | 1.85(-) | >3.22(-) |

In Table 2, (-) shows no cytotoxicity.

PHARMACOLOGICAL EFFICACY TESTING - 3

In Vivo Anti-HSV-1 Action of 1-(o-chlorophenyl)piperazine hydrochloride

Male BALB/c mice of five weeks of age were intraperitoneally infected with 322 PFU (plaque forming unit, $10LD_{50}$) of HSV-1 (Miyama strain). Twenty-one (21) days later, the survival ratios and mean survival days were evaluated. The test compound = 1-(o-chlorophenyl)piperazine hydrochloride= was dissolved in a physiological saline solution, and intraperitoneally administered the animals at 5 and 20 mg/kg for five days just after virus incubation.

The results are shown Table 3. Significant increases in the survival rattios and mean survival days were observed in the compound treated group.

Table 3

In Vivo Anti-HSV-1 Activities of 1-(o-chlorophenyl)piperazine hydrochloride

| Doses | Survival Ratios after 21 days (%) | Mean Survival Days |
|---|---|---|
| 0 mg/kg | 0 | 6.2 |
| 5 mg/kg | 17 | 9.3 |
| 20 mg/kg | 33 | 12.7* |

*: P<0.05 (U Test)

PHARMACOLOGICAL EFFICACY TESTING - 4

In Vivo Anti-HSV-1 Activities of 4-(2-aminoethyl)-1-(o-methoxyphenyl)piperazine

Male BALB/c mice of six weeks of age were intraperitoneally infected with 322 PFU (plaque forming unit, $10LD_{50}$) of HSV-1 (Miyama strain). Twenty-one (21) days later, the survival ratios and mean survival days were evaluated. The test compound = 4-(2-aminomethyl)-1-(0-methoxyphenyl)piperazine = was dissolved in 5% arabic gum/physiological saline solution, and intraperitoneally administered to the animals at 10 and 20 mg/kg for five days just after virus incubation.

The results are shown Table 4. Significant increases in the survival rattios and mean survival days were observed in the compound treated group.

Table 4

In Vivo Anti-HSV-1 Activities of 4-(2-aminoethyl)-1-(o-methoxyphenyl)piperazine

| Doses | Survival Ratios after 21 days (%) | Mean Survival Days |
|---|---|---|
| 0 mg/kg | 0 | 6.9 |
| 5 mg/kg | 70 | 17.5* |
| 20 mg/kg | 50 | 10.9* |

*: P<0.05 (U Test)

PHARMACOLOGICAL EFFICACY TESTING - 5

In Vivo Anti-HSV-1 Activities of (3α-16α)-eburnamonine-O-[2-hydroxy-3-[4-(o-methoxyphenyl)-1-piperazinyl]propyl]oxime trihydrochloride

Male BALB/c mice of six weeks of age were intraperitoneally infected with 322 PFU (plaque forming unit, $10LD_{50}$) of HSV-1 (Miyama strain). Twenty-one (21) days later, the survival ratios and mean survival days were evaluated. The test compound = (3α,16α)-eburnamonine-0-[2-hydroxy-3-{4-(o-methoxyphenyl)-1-piperazinyl]propyl]oxime trihydrochloride = was dissolved in a physiological saline, and intraperitoneally administered to the animals at 5, 10, 20 and 40 mg/kg for five days just after virus incubation.

The results are shown Table 5. Significant increases in the survival rattios and mean survival days were observed in the compound treated group.

Table 5

In Vivo Anti-HSV-1 Action of (3α,16α)-eburnamonine-O-[2-hydroxy-3-[4-(o-methoxyphenyl)-1-piperazinyl]propyl]oxime trihydrochloride

| Doses | Survival Ratios after 21 days (%) | Mean Survival Days |
|---|---|---|
| 0 mg/kg | 0 | 6.9 |
| 5 mg/kg | 50 | 15.4* |
| 10 mg/kg | 60 | 15.4* |
| 20 mg/kg | 50 | 16.4* |
| 40 mg/kg | 50 | 15.5* |

*: $P<0.05$ (U Test)

PREPARATION EXAMPLES

Preparation Example 1 (Ointment)

According to the following recipe, ointment was prepared conventionally and packed in an aluminum tube.

| | |
|---|---|
| 1-(o-chlorophenyl)piperazine hydrochloride | 3g |
| White vaseline | suitable amount |
| | Total: 100 g |

Preparation Example 2 (Oral Ointment)

According to the following recipe, ointment was prepared conventionally and packed in an aluminum tube.

| | |
|---|---|
| 1-(o-methoxyphenyl)piperazing hydrochloride | 0.3 g |
| Carboxylmethycellulose (Na) | 3.1 g |
| Liquid paraffin | 3.1 g |
| White vaseline | 1.2 g |
| Base | suitable amount |
| | Total: 100g |

Preparation Example 3 (Capsule)

According to the following recipe, a capsule was prepared conventonally.

| | |
|---|---|
| Compound 9 | 100 mg |
| Magnesium stearate | 5 mg |
| Lactose | suitable amount |

Compound 9: 4-(aminoethyl)-1-(o-methoxyphenyl)piperazine

Preparation Example 4 (Tablet)

According to the following recipe, the following components were blended with a vehicle and tableted conventionally.

| | |
|---|---|
| Compound 10 | 100 mg |
| Sodium lauryl sulfate | 10 mg |
| Magnesium stearate | 5 mg |
| Polyvinyl pyrrolidone K30 | 11 mg |
| Carboxymethylcellulose (Ca) | 7 mg |
| Lactose | 60 mg |
| Corn starch | suitable amounts |
| | Total: 100 mg |

Compound 10: (3α,16α)-ebrunamonine-O-[2-hydroxy-3-[4-(o-methoxyphenyl)-1-piperazinyl]propyl]oxime (trihydrochloride

The modified or unmodified phenylpiperazine, phenylpiperidine, phenylpyrrolidine, tetrahydroimidazole, phenylhomopiperazine, phenylaziridine, phenylazetidine, phenyldiazetidine or phenylperhydroazepine according to the invention had an excellent antiviral action in itself, and provided an excellent effect on the prevention and treatment of infections by various DNA viruses, RNA viruses and retroviruses. Further, it is possible to provide some known compounds with an antiviral activity, or enhance the antiviral activities thereof, by introducing into them modified or unmodified phenylpiperazine, phenylpiperidine, phenylpyrrolidine, tetrahydroimidazole, phenylhomopiperazine, phenylaziridine, phenylazetidine, phenyldiazetidine or phenylperhydroazepine according to the invention as substituents. Still further, these compounds, because of being quite different in chemical structure from existing antiviral agents, are expected to be efficacious against infections by viruses resistant to the existing antiviral agents.

## Claims

1. An antiviral agent containing as a main component a compound represented by the following general formula (I):

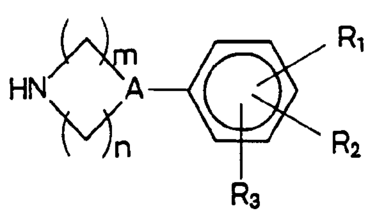

(I)

wherein,

$R_1$, $R_2$ and $R_3$ simultaneously or independently represent a hydrogen atom, an amino group, an alkylamino group that may have a substituent, an acylamino group, an alkyl group that may have a substituent, a hydroxy group, an alkyloxy group that many have a substituent, a halogeno group, a carboxy group, an alkylcarbonyl group that may have a substituent, an alkoxycarbonyl group that may have a substituent, a carbamoyl group that may have a substituent, a nitro group, a cyano group, a thiol group, an alkylthio group that may have a substituent, a phenyl group that may have a substituent or a heterocyclic ring;

A is a nitrogen atom or a methyne group;
m is 0 or a natural number; and
n is a natural number;

or a salt thereof.

2. An antiviral agent as claimed in Claim 1, wherein said substituent is selected from amino, halogeno, nitro, cyano, thiol, acyl and carbamoyl groups, and said heterocyclic ring is selected from pyridine, piperidine, pyrazine, pyrrole, pyrrolidine, oxazole, imidazole, morpholine, diazole, tetrazole, thiazole or thiadiazole.

3. An antiviral agent as claimed in Claim 1, wherein said compound is selected from:
1-phenylpiperazine,
1-(o-chlorophenyl)piperazine,
1-(m-chlorophenyl)piperazine,
1-(p-chlorophenyl)piperazine,
1-(o-fluorophenyl)piperazine,
1-(m-fluorophenyl)piperazine,
1-(p-fluorophenyl)piperazine,
1-(o-methoxyphenyl)piperazine,
1-(m-methoxyphenyl)piperazine,
1-(p-methoxyphenyl)piperazine,
1-(o-methylphenyl)piperazine,
1-(m-methylphenyl)piperazine,
1-(p-methylphenyl)piperazine,
1-(o-trifluoromethylphenyl)piperazine,
1-(m-trifluoromethylphenyl)piperazine,
1-(p-trifluoromethylphenyl)piperazine,
1-(o-nitorphenyl)piperazine,
1-(m-nitrophenyl)piperazine,
1-(p-nitrophenyl)piperazine,
1-(2,6-dichlorophenyl)piperazine
1-phenylpiperidine, and their salts.

4. An antiviral agent containing as a main component 4-(2-aminoethyl)-1-(o-methoxyphenyl)piperazine, (3α,16α)-ebrunamonine-O-[2-[4-(o-methoxyphenyl)-1-piperazinyl]ethyl]oxime and (3α,16α)-eburnamonine-0-[2-hydroxy-3-[4-(o-methoxyphenyl)-1-piperazinyl]oxime or a salt thereof.

5. A pharmaceutical composition comprising an antiviral compound as defined in any one of Claims 1 to 4 and an absorption promoter.

6. A pharmaceutical composition as claimed in Claim 5, wherein said absorption promoter is a polyoxyethylene higher alkyl ether or a surface active agent.

7. Use of a compound as defined in any one of Claims 1 to 4 for the prevention and treatment of infections caused by various DNA viruses, RNA viruses and retroviruses; herpes viruses; or influenza viruses.

8. Use of a compound as defined in any one of Claims 1 to 4 in the manufacture of a medicament for prevention and treatment of infections caused by various DNA viruses, RNA viruses and retroviruses; herpes viruses; or influenza viruses.

9. A compound endowed with antiviral activity, or having its existing antiviral activity enhanced, by the introduction thereinto, as a substituent, of a compound as defined in any one of Claims 1 to 4.

10. The features as herein disclosed, or their equivalents, in any novel, patentable selection.

European Patent Office

# EUROPEAN SEARCH REPORT

Application Number

EP 92 30 9622

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X<br>Y | EP-A-0 156 433 (JANSSEN PHARMACEUTICA N.V.)<br>* claims 2,3; examples 38,41,43,53 * | 1,2,7-10<br>3-6 | A61K31/495<br>A61K31/445 |
| X<br><br><br><br>Y | CHEMICAL ABSTRACTS, vol. 87, no. 23, 5 December 1977, Columbus, Ohio, US; abstract no. 184549, page 617 ;<br>* abstract *<br>& JP-A-7 731 082 (AJINOMOTO CO., INC.) 9 March 1977 | 1,2,7-10<br><br><br><br>3-6 | |
| X | EP-A-0 168 197 (SANWA KAGAKU KENKYUSHO CO. LTD.)<br>* claims 5,6; examples 5,6; tables 1-3 * | 4,9,10 | |
| X | EP-A-0 394 553 (DAIICHI PHARMACEUTICAL CO. LTD.)<br>* page 2, line 53; example 1 * | 1,2,7-10 | |
| X | EP-A-0 422 485 (BAYER AG)<br>*page 10, line 5 and 10; tabel 1, e.g. page 37, line 25 and 30; claims 1 and 13* | 1,2,7-10 | |
| A | EP-A-0 284 552 (CIBA-GEIGY AG)<br>*pages 1, 9, 11, 12* | 1-10 | TECHNICAL FIELDS SEARCHED (Int. Cl.5 )<br>A61K |

The present search report has been drawn up for all claims

| Place of search | Date of completion of the search | Examiner |
|---|---|---|
| MUNICH | 03 DECEMBER 1992 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)